# EUROPÄISCHE PATENTSCHRIFT

(11) **EP 3 452 576 B1**
(45) Veröffentlichungstag und Bekanntmachung des Hinweises auf die Patenterteilung: **28.04.2021**
(21) Anmeldenummer: 18713568.6
(22) Anmeldetag: 02.03.2018
(51) Int. Cl.: C12M 1/26, C12M 1/34, C12M 1/36, B05B 7/24, B05B 9/03, B05C 11/10

(54) **VERFAHREN UND VORRICHTUNG ZUR DOSIERUNG UND AUFBEWAHRUNG VON FLÜSSIGKEITEN MITTELS PERMANENT OFFENER BEHÄLTER**
METHOD AND DEVICE FOR METERING AND STORING LIQUIDS BY MEANS OF PERMANENTLY OPEN CONTAINERS
PROCÉDÉ ET DISPOSITIF POUR LE DOSAGE ET LA CONSERVATION DE LIQUIDES À L'AIDE DE RÉCIPIENTS OUVERTS EN PERMANENCE

(30) Priorität: 03.03.2017 DE 102017002046
(43) Veröffentlichungstag der Anmeldung: 13.03.2019
(73) Patentinhaber: Aquila Biolabs GmbH, 52499 Baesweiler (DE)
(72) Erfinder: HERZOG, Konrad, 52499 Baesweiler (DE); FRANK, David, 52499 Baesweiler (DE); PRESSER, Carsten, 52499 Baesweiler (DE)
(74) Vertreter: Roth, Andy Stefan
(86) Internationale Anmeldenummer: PCT/EP2018/055176
(87) Internationale Veröffentlichungsnummer: WO 2018/158431

(56) Entgegenhaltungen:
- EP-A1- 2 492 658
- WO-A1-2005/099906
- DE-A1-102011 078 855
- DE-B3-102016 000 997
- JP-A- H02 184 370

## Beschreibung

### Technisches Gebiet

Die Erfindung betrifft ein Verfahren und eine Vorrichtung zur Dosierung und Aufbewahrung von Flüssigkeiten mittels permanent offener Behälter. Die Erfindung ist insbesondere anwendbar bei der Dosierung und Aufbewahrung von Flüssigkeiten im Rahmen biologischer und chemischer Prozesse, welche über Stunden oder Tage ablaufen. Weiterhin ist sie insbesondere überall dort anwendbar, wo die zu dosierende oder aufzubewahrende Flüssigkeit sowie ihr Behälter Temperaturänderungen oder Erschütterungen ausgesetzt ist.

Permanent offene Behälter werden häufig zur Dosierung von Flüssigkeiten in den verschiedensten Bereichen biologischer oder chemischer Arbeiten und Prozesse eingesetzt. Einige der am weitesten verbreiteten Behältertypen sind dabei insbesondere Spritzen und Pipetten. Diese zeichnen sich durch mindestens eine permanent offene Dosieröffnung aus und besitzen die charakteristische Eigenschaft, dass in ihnen enthaltene Flüssigkeit auch dann im Behälter verbleibt, wenn mindestens eine Dosieröffnung unter die Flüssigkeit gebracht wird. Ursächlich dafür ist ein Gleichgewicht aus Umgebungsluftdruck, hydrostatischem Druck der Flüssigkeit, Druck im Behälter, sowie Oberflächen- und Kapillareffekten.

Dieses Gleichgewicht ist jedoch durch äußere Faktoren beeinflussbar, was zu einer nachteiligen Änderung der Flüssigkeitsverteilung führen kann, insbesondere durch Verlust von Flüssigkeit durch mindestens eine Behälteröffnung oder durch Änderung der Flüssigkeitsverteilung innerhalb des Behälters (z.B. durch Einsaugung). Ersteres führt zu unerwünschten Dosiervorgängen und zweiteres führt zu einem nachteiligen Verlust an Dosierpräzision. Bedeutsame äußere Einflussfaktoren sind dabei Erschütterungen oder anderweitige ungewollte Beschleunigungen der Flüssigkeit hin zur Dosieröffnung, sowie thermodynamische oder chemische Prozesse, welche eine Volumen- oder Druckänderung innerhalb des Behälters hervorrufen. Auch Änderungen der Oberflächeneigenschaften des Behälters oder seines Inhalts sind als Einflussfaktoren zu erwähnen.

### Stand der Technik

Die JP H02 184370 A offenbart eine Spritze mit einem Spritzenkörper, welcher teilweise mit einer Flüssigkeit gefüllt ist. Innerhalb des Spritzenkörpers ist eine Gasphase ausgebildet. Durch äußere Einflüsse hervorgerufene Druckschwankungen werden überwacht und entsprechend gegengeregelt.

Die DE 10 2011 078855 A1 offenbart eine Vorrichtung zum definierten und dosierten Zuführen von Flüssigkeiten zu einer Lösung. Eine erste Pumpvorrichtung ist zum Dosieren und Definieren einer zuzuführenden Menge der Flüssigkeit vorgesehen.

### Aufgabenstellung

Es ist Aufgabe der vorliegenden Erfindung, ein Verfahren anzugeben, mittels dessen die Dosierung und Aufbewahrung von Flüssigkeiten in permanent offenen Behältern robust erfolgen kann. Die der Erfindung zugrundeliegende Aufgabe wird gelöst durch ein Verfahren nach Anspruch 1 sowie eine Vorrichtung nach Anspruch 12; bevorzugte Ausgestaltungen ergeben sich aus den Unteransprüche sowie der Beschreibung.

### Definitionen

Zur Sicherstellung der Klarheit einiger in der Beschreibung verwendeter Begriffe, werden diese nachfolgend und im Verlauf der Beschreibung definiert und erläutert.

Erfindungsgemäß ist ein Behälter ein Gefäß, welches geeignet ist, Flüssigkeit aufzubewahren oder zu dosieren. Behälter können gegenüber ihrer Umwelt geschlossen oder offen sein. Erfindungsgemäße Behälter zeichnen sich insbesondere durch mindestens eine permanente Öffnung aus, welche die Dosierung von Flüssigkeit aus dem Behälter erlaubt. In vielen Fällen und insbesondere wenn die Dosierung durch fallende Tropfen erfolgt, ist die permanent offene Öffnung gleichzeitig der tiefste Punkt des Behälters, sodass sich bei befülltem Behälter permanent Flüssigkeit über der Öffnung befindet. Erfindungsgemäß kann ein Behälter auch aus mehreren Teilen bestehen. Ein Behälter endet entlang seiner Wände sowie an Öffnungen entlang der kleinsten oder direktesten Fläche zum Verschluss dieser Öffnung.

Einflussfaktoren im Sinne der Erfindung sind alle physikalischen, chemischen oder biologischen Phänomene oder Effekte, welche insbesondere aber nicht ausschließlich zu einer Veränderung der Verteilung oder des Volumens der Flüssigkeit im Behälter, zu einer Veränderung des Bewegungszustands der Flüssigkeit im Behälter oder zu einer Änderung der Druckverhältnisse im Behälter führen.

Reaktionen im Sinne der Erfindung sind alle Verfahren oder Prozesse, die geeignet sind, den stabilen Zustand des erfindungsgemäßen Systems zu erhalten, wiederherzustellen oder den aktuellen Zustand dem stabilen Zustand zumindest anzunähern. Reaktionen sind parametrisierbar und können überwacht und geregelt werden. Reaktionen umfassen immer eine definierte Anzahl an Aktionen, sie haben daher einen Anfang und ein Ende. Typische Reaktionen umfassen insbesondere aber nicht ausschließlich das Öffnen oder Schließen eines Ventils, das Fördern oder die Änderung der Förderrichtung einer Pumpe sowie das Ändern von Temperaturen durch Aufheizen oder Abkühlen. Im Anschluss an eine oder während einer Reaktion erfolgt nach einer definierten oder undefinierten Zeit eine Aufnahme und Überprüfung des aktuellen Systemzustands, um die Reaktion und ihre Folgen zu beurteilen.
Verfahren zur Dosierung und Aufbewahrung von Flüssigkeiten mittels permanent offener Behälter werden im Sinne der Erfindung durch mindestens eine Regelungseinheit gesteuert. Insofern sind Regelungseinheiten alle Vorrichtungen, welche geeignet sind, in Abhängigkeit von erfassten Druckwerten oder Werten weiterer Parameter Reaktionen zur Erhaltung oder Wiederherstellung des stabilen Zustands eines erfindungsgemäßen Systems einzuleiten oder zu steuern. Je nach eingesetztem Verfahren sind Regelungseinheiten im Sinne der Erfindung insbesondere aber nicht ausschließlich Operationsverstärker, Komparatoren, PID-Regler, Schmitt-Trigger und Rechner. Als Rechner zählt jede elektronische Vorrichtung, die Daten (insbesondere arithmetische und logische) speichern und auf der Grundlage programmierbarer Vorschriften verarbeiten kann. Als Rechner im Sinne der Erfindung gelten insbesondere aber nicht ausschließlich Mikrocontroller, Mikroprozessoren, System-on-a-Chip Rechnern (SoC), PCs und Server.

Flüssigkeiten im Sinne der Erfindung sind reine oder gemischte Substanzen, die keine Gase sind und fluidische Eigenschaften aufweisen. Flüssigkeiten im Sinne der Erfindung sind somit insbesondere aber nicht ausschließlich Flüssigphasen reiner Stoffe, Lösungen, Emulsionen, Dispersionen, Schlämme, Suspensionen und Schäume. Aufgrund ihres makroskopisch mit Fluiden vergleichbarem Verhalten werden auch feine, rieselfähige Pulver und Pulvergemische unter dem Begriff Flüssigkeit zusammengefasst.

Erfindungsgemäß erfolgt die Dosierung von Flüssigkeiten aus Behältern. Dosierung im Sinne der Erfindung bezeichnet jeden Prozess, der geeignet ist, Flüssigkeit gezielt aus einem Behälter abzugeben. Dosierungen werden durch mindestens eine gesteuerte Fluidtransportvorrichtung vorgenommen, wobei diese entweder passiv (z.B. Öffnen/Schließen eines Ventils) oder aktiv (z.B. Fluidförderung durch Pumpe) arbeitet. Dosierung nach Bedarf umfasst im Sinne der Erfindung sämtliche gesteuerten Dosierungsprozesse, insbesondere solche, die durch Nutzer vorprogrammiert wurden (z.B. zeitabhängige Dosierungsprofile), die durch direkte Nutzerinteraktionen (z.B. Knopfdruck) erfolgen oder die über die Erfassung bestimmter Parameter oder Prozessgrößen (z.B. Zellzahl, pH, Schaum, Produktmenge, Eduktmenge, etc.) geregelt werden. Dosierschritte sind diskrete Dosierungen, beispielsweise ein einzelner Tropfen, eine Umdrehung des Pumpenmotors oder ein Öffnungs- und Schließvorgang eines Ventils.

Erfindungsgemäß bezeichnet ein System die Gesamtheit mehrerer sich in einer gemeinsamen Umgebung befindlichen Komponenten, insbesondere das System aus erfindungsgemäßer Vorrichtung mit Flüssigkeit. Jedes System befindet sich zu jeder Zeit in einem bestimmten Zustand oder Systemzustand, welcher charakterisiert wird durch verschiedene, teils ortsabhängige Parameter, insbesondere aber nicht ausschließlich durch Druck, Temperatur, Volumen, Beschleunigung, Position und Lage.

Als stabiler Zustand im Sinne der Erfindung gelten all jene Zustände, bei denen keine Abgabe von Flüssigkeit aus dem Behälter erfolgt und aus denen heraus für das Anwendungsfeld hinreichend präzise Dosierungen möglich sind. Für hinreichend präzise Dosierungen sind insbesondere das hinreichend präzise Wissen um aktuelle Totvolumina und Flüssigkeitsverteilungen oder Füllstände relevant. Dementsprechend sind alle anderen Zustände, insbesondere solche, die einen Verlust von Flüssigkeit oder Dosierpräzision verursachen, unstabile Zustände im Sinne der Erfindung.

Sämtliche mindestens einen bestimmten Zustand charakterisierenden Parameter gelten neben dem Druck als weitere Parameter im Sinne der Erfindung und können als solche durch geeignete Sensoren erfasst und in erfindungsgemäße Verfahren einbezogen werden. Als weitere Parameter gelten auch Nutzerinteraktionen sowie Steuerbefehle oder Messdaten anderer Vorrichtungen. Im Sinne der Erfindung können sämtliche erfassbaren Parameter einzeln oder mehrfach, parallel oder sequentiell sowie automatisch oder ereignisgekoppelt erfasst werden. Aus einzelnen oder mehreren einen bestimmten Zustand charakterisierenden Parametern oder aus von ihnen abgeleiteten Werten können im Sinne der Erfindung Modelle erzeugt und angepasst werden, welche zur vollständigen oder teilweisen Beschreibung und Evaluierung des bestimmten Zustandes nutzbar sind.

Erfindungsgemäß erfolgt die Erfassung des Drucks oder der Änderung des Drucks als absoluter oder differentieller Wert über Drucksensoren. Drucksensoren im Sinne der Erfindung sind neben klassischen Piezo-, Resistiv-, oder sonstigen MEMS-Sensoren insbesondere auch solche Sensoren und Soft-Sensoren, die mindestens einen vom Druck verschiedenen Parameter erfassen und aus ihm und gegebenenfalls bekannten Randbedingungen den Druck, die Druckänderung oder andere mit diesen Parametern korrelierende Werte berechnen. Ein Beispiel dafür ist die Berechnung des Drucks über Gasgleichungen aus Temperaturmesswerten und bekannten oder gemessenen Volumina.

### Lösung

Erfindungsgemäß erfolgt eine Überwachung der Druckverhältnisse im Behälter sowie durch eine geeignete Reaktion auf die erfassten Druckänderungen. Grundlage der Erfindung ist dabei die Erkenntnis, dass jeder Einflussfaktor, welcher eine nachteilige Verteilungsänderung der Flüssigkeit im Behälter bedingt, eine Änderung der Druckverhältnisse im Behälter hervorruft. Durch Erfassung dieser Druckänderung wird dann eine geeignete Reaktion eingeleitet, welche der Veränderung der Flüssigkeitsverteilung im Behälter entgegenwirkt. Somit wird unabhängig von thermodynamischen, mechanischen oder chemischen Einflussfaktoren auch bei permanent geöffnetem Behälter einem Verlust an Flüssigkeit oder Dosierpräzision entgegengewirkt.

Erfindungsgemäß wird der Druck innerhalb des Behälters durch mindestens einen Drucksensor überwacht, welcher die durch ihn erfassten Druckdaten oder aus ihnen abgeleitete Werte an mindestens eine Regelungseinheit übermittelt, welche mindestens eine geeignete Reaktion einleitet, um der Veränderung der Flüssigkeitsverteilung im Behälter entgegenzuwirken.

Es ist zwischen dem Flüssigkeitsreservoir und der Dosiervorrichtung, also zwischen dem Flüssigkeitsreservoir einserseits und dem Drucksensor und/oder der Fluidtransportvorrichtung andererseits, eine Sterilitätsbarriere eingebracht (z.B. ein Sterilfilter), sodass die Sterilitätsbedingungen im Flüssigkeitsreservoir aufrechterhalten werden können.

In einigen Ausführungsformen der Erfindung erfolgt die Erfassung von Druckänderungen durch mehrere Drucksensoren an verschiedenen Positionen.

In vorteilhafter Ausgestaltung der Erfindung befindet sich nicht nur Flüssigkeit, sondern auch eine gasförmige Komponente im Behälter. Die Gasphase des Behälterinhalts ist in vorteilhafter Ausgestaltung kompressibler als die Flüssigphase. Dies erlaubt insbesondere die Erfassung von Druckänderungen nur über die Gasphase, sodass in vorteilhafter Ausgestaltung der Erfindung kein direkter Kontakt zwischen der Flüssigkeit im Behälter und mindesten einem Drucksensor besteht.

In vorteilhafter Ausgestaltung der Erfindung erfolgt die gezielte Bewegung oder Umverteilung der Flüssigkeit im Behälter über die Bewegung einer Gasphase, insbesondere Luft. Erfindungsgemäß ist die gezielte Bewegung oder Umverteilung der Flüssigkeit im Behälter sowohl Teil des normalen Dosierungsprozesses als auch des Reaktionsprozesses auf erfasste Druckänderungen im Behälter.

In vorteilhafter Ausgestaltung der Erfindung und insbesondere, wenn eine Gasphase zur gezielten Bewegung oder Umverteilung der Flüssigkeit im Behälter genutzt wird, ist der Behälter aus mindestens zwei Bestandteilen zusammengesetzt, welche sich in einigen Ausführungsformen der Erfindung durch den Anwender der Vorrichtung verbinden oder trennen lassen. In vorteilhafter Ausgestaltung der Erfindung setzt sich der mindestens zweiteilige Behälter zumindest aus einem Flüssigkeitsreservoir und einer Dosiervorrichtung zusammen, wobei es Aufgabe des Flüssigkeitsreservoirs ist die zu dosierende Flüssigkeit über den gesamten Dosierprozess aufzubewahren und wobei die Dosiervorrichtung die kontrollierte Abgabe von Flüssigkeit aus dem Flüssigkeitsreservoir im Rahmen mindestens eines Dosierschritts ermöglicht.

In vorteilhafter Ausgestaltung der Erfindung sind Flüssigkeitsreservoir und Dosiervorrichtung gasdicht miteinander verbunden.

Durch erfindungsgemäße Verwendung eines Behälters aus mehreren Bestandteilen in Kombination mit der indirekten Bewegung oder Umverteilung der Flüssigkeit mittels einer Gasphase wird in vorteilhafter Ausgestaltung der Erfindung erreicht, dass insbesondere durch Kompartimentierung oder durch Verwendung geeigneter Materialien im Flüssigkeitsreservoir Bedingungen aufrechterhalten werden können, die außerhalb des Flüssigkeitsreservoirs nicht aufrechterhaltbar sind. Eine in biologischen Applikationen besonders wichtige Bedingung ist dabei die Sterilität.

In einigen Ausführungsformen der Erfindung ist die Dosiervorrichtung mehrfach verwendbar, während das Flüssigkeitsreservoir nur einmalig eingesetzt werden kann und vor jedem neuen Dosierprozess ausgetauscht werden muss. Vorteilhaft ist dies insbesondere im Bereich biologischer Applikationen, da hier ein Einweg-Flüssigkeitsreservoir bereits vorsterilisiert durch den Nutzer der erfindungsgemäßen Vorrichtung eingesetzt wird und somit nutzerfreundlich die notwendigen Sterilitätsbedingungen im Flüssigkeitsreservoir aufrechterhalten werden können.

In Ausführungsformen, die eine Kompartimentierung des Flüssigkeitsbehälters durch geeignete Barriere-Vorrichtungen aufweisen, erfolgt in vorteilhafter Ausgestaltung der Erfindung die Erfassung mindestens eines Druckunterschiedes im Behälter durch mindestens einen Drucksensor derart, dass der Drucksensor hinter der Barriere und damit außerhalb des Flüssigkeitsreservoirs lokalisiert ist.

In vorteilhafter Ausgestaltung der Erfindung erfolgt die Erfassung von Druckänderungen durch mindestens einen Drucksensor mit derart hohen Messfrequenzen (z.B. >10Hz, >100Hz, >1000Hz), sodass auch auf entsprechend schnelle Druckänderungen, wie sie beispielsweise durch Erschütterungen beim Transport oder der Installation befüllter erfindungsgemäßer Vorrichtungen auftreten, adäquat reagiert werden kann.

In vorteilhafter Ausgestaltung der Erfindung erfolgt die Positionierung mindestens eines Drucksensors derart, dass er nicht durch Bewegungen, welche keinen nachteiligen Einfluss auf die Verteilung der Flüssigkeit im Behälter haben, beeinflusst oder mit Hintergrundsignalen beaufschlagt wird. Insbesondere aber nicht ausschließlich erfolgt in vorteilhafter Ausgestaltung der Erfindung für geschüttelte Systeme die Positionierung mindestens eines Drucksensors derart, dass das sensitive Element des Sensors (z.B. Membran, Widerstandsbrücke, Piezokristall, etc.) mit seiner sensorisch vorteilhaften mechanischen Auslenkungsrichtung orthogonal zur Schüttelebene positioniert ist.

In einigen Ausführungsformen der Erfindung werden neben dem Druck auch weitere Parameter erfasst und überwacht, welche geeignet sind, Einflussfaktoren und ihr direktes oder indirektes Wirken auf die Verteilung der Flüssigkeit im Behälter zu identifizieren und geeignete Reaktionen einzuleiten. Derartige, zusätzlich zum Druck im Behälter erfassbare Parameter sind insbesondere aber nicht ausschließlich der Umgebungsdruck des Behälters, die Temperatur des Behälters, seines Inhalts oder einzelner Bestandteile seines Inhalts, der Bewegungs-, Beschleunigungs-, Positions-, oder Lagezustand des Behälters, seines Inhalts oder einzelner Bestandteile seines Inhalts, der Befüllungszustand des Behälters sowie die Verteilung der Flüssigkeit im Behälter. Erfindungsgemäß zu diesem Zweck einsetzbare Sensoren sind insbesondere alle Arten von Drucksensoren, Temperatursensoren, Beschleunigungs-, Lage-, Positions- und Drehratensensoren sowie optische, akkustische, kapazitive und resistive Füllstandsensoren.

In einigen Ausführungsformen der Erfindung wird aus den erfassten Druckwerten sowie aus gegebenenfalls weiteren verfügbaren Werten das Volumen der Flüssigkeit im Behälter errechnet.

In einigen Ausführungsformen der Erfindung werden erfasste Druckwerte und gegebenenfalls weitere verfügbare Werte genutzt, um Vorhersagen über zukünftig zu erwartende Flüssigkeitsverteilungen zu tätigen. Vorteilhaft ist dies insbesondere, aber nicht ausschließlich, um präventive Reaktionen einzuleiten, welche insbesondere die nachteilige Wirkung von sich schnell ändernden Einflussfaktoren auf die Verteilung der Flüssigkeit im Behälter vorrausschauend abmildern oder ihnen vorbeugen können. In vorteilhafter Ausgestaltung der Erfindung werden zu diesem Zweck Vorhersagemethoden wie Kalman-Filter oder anderweitige bayessche Schätzverfahren, sowie Regressions- und Extrapolationsmethoden eingesetzt.

In vorteilhafter Ausgestaltung der Erfindung umfasst die Reaktion auf mindestens eine erfasste Druckänderung im Behälter die vollständige oder zumindest annähernde Wiederherstellung des vor der Druckänderung im Behälter vorherrschen Drucks, wodurch einer Veränderung der Flüssigkeitsverteilung entgegengewirkt oder diese rückgängig gemacht wird. Derartige Reaktionen umfassen insbesondere, aber nicht ausschließlich, das Einbringen oder Herausbefördern von Fluiden in den oder aus dem Behälter, sowie das Aufheizen oder Abkühlen des Behälters, seines Inhalts oder einzelner Bestandteile seines Inhalts.

In einigen Ausführungsformen der Erfindung erfolgt die Reaktion auf mindestens eine zukünftig zu erwartende Druckänderung im Reaktor oder Beschleunigung der Flüssigkeit präventiv, insbesondere um auch das Wirken solcher Einflussfaktoren abschwächen oder eliminieren zu können, welche derart schnelle Flüssigkeitsverteilungs- und Druckänderungen hervorrufen, dass diese nicht mehr durch mindestens einen Drucksensor erfassbar sind und somit über die Regelungseinheit keine adäquate rückgekoppelte Reaktion mehr eingeleitet werden kann.

In einigen Ausführungsformen der Erfindung erfolgt Erfassen des Drucks und/oder das Einleiten der Reaktion und/oder das Abgeben der Flüssigkeit erfolgt, während das Gefäß geschüttelt wird. Das erfindungsgemäße Verfahren wird folglich insbesondere zeitlich parallel und fortlaufend zum Vorgang im Gefäß durchgeführt. Die Vorrichtung bzw. der Behälter mitsamt der Dosiervorrichtung werden insbesondere gemeinsam mit dem Gefäß geschüttelt.

Insbesondere verschließt die Vorrichtung das Gefäß, wobei insbesondere ein Deckel des Behälters auch das Gefäß abdeckt.

Die vorliegende Erfindung wird anhand der Figuren und Ausführungsbeispiele näher erläutert.

### Ausführungsbeispiele und Figuren

Figur 1, zeigt ein schematisches Flussdiagramm des erfindungsgemäßen Verfahrens.
Figur 2, zeigt eine schematische Blockdarstellung des erfindungsgemäßen Verfahrens unter Verwendung einer erfindungsgemäßen Vorrichtung.
Figur 3, zeigt ein schematisches Flussdiagramm einer Ausführungsform des erfindungsgemäßen Verfahrens mit zusätzlicher Erfassung weiterer Parameter sowie mit Vorhersage- und Analyseverfahren und präventiver Reaktion.
Figur 4, zeigt eine schematische Darstellung einer erfindungsgemäßen Vorrichtung zur Durchführung des erfindungsgemäßen Verfahrens zur Kultivierung von Zellen.

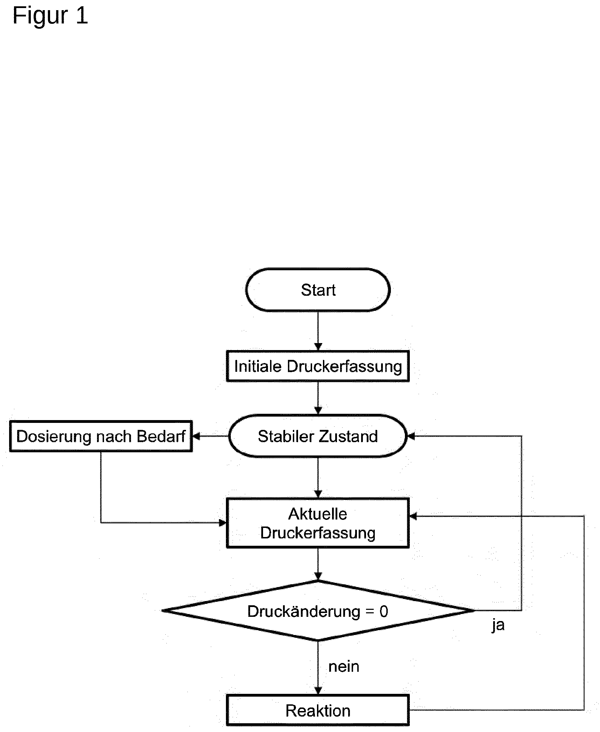

Figur 1 zeigt ein schematisches Flussdiagramm des erfindungsgemäßen Verfahrens. Erfindungsgemäße Verfahren werden insbesondere durch erfindungsgemäße Vorrichtungen zur Dosierung und Aufbewahrung von Flüssigkeiten ausgeführt. Zu Beginn des erfindungsgemäßen Verfahrens hat die Vorrichtung einen Start-Zustand. Üblicherweise, aber nicht notwendigerweise, ist dieser Start-Zustand der Zustand der Vorrichtung direkt nach der Befüllung mit Flüssigkeit. Aus dem Startzustand erfolgt mindestens eine initiale Druckerfassung, die mit dem Übergang in einen und der Festsetzung eines stabilen Zustands abgeschlossen ist. Je nach äußeren Bedingungen kann die initiale Druckerfassung innerhalb weniger Sekunden abgeschlossen sein, oder aber auch deutlich länger andauern. Weiterhin kann, je nach Ausführungsform der Erfindung, die initiale Druckerfassung sowohl automatisch mit Beginn des erfindungsgemäßen Verfahrens, oder aber als Folge einer Nutzerinteraktion starten.

Nach Abschluss der Initialisierungsphase befindet sich das System aus erfindungsgemäßer Vorrichtung und dem Behälterinhalt in einem stabilen Zustand. In vorteilhafter Ausgestaltung der Erfindung erfolgen alle weiteren Verfahrensschritte entweder aus dem stabilen Zustand heraus oder haben das Ziel, den stabilen Zustand zu erhalten oder wiederherzustellen. Erfindungsgemäß erfolgt je nach Bedarf die Dosierung von Flüssigkeit aus dem Behälter aus dem stabilen Zustand heraus.

Erfindungsgemäß wird in regelmäßigen oder unregelmäßigen Abständen überprüft, ob Abweichungen vom stabilen Zustand aufgetreten sind. Dies erfolgt durch mindestens eine aktuelle Druckerfassung, auf deren Grundlage die Druckänderung zwischen aktuellem Druck und dem Druck des stabilen Zustands ermittelt wird. Ist keine Druckänderung erfolgt, so befindet sich das System weiterhin im stabilen Zustand. Ist jedoch eine Druckänderung erfolgt, so wird erfindungsgemäß eine Reaktion eingeleitet, welche insbesondere aber nicht ausschließlich dem Zweck der Wiederherstellung des stabilen Zustands dient und damit eine nachteilige Veränderung der Flüssigkeitsverteilung im Behälter mit Fehldosierung oder Dosierungspräzisionsverlust verhindert oder rückgängig macht.

Erfindungsgemäß erfolgt im Anschluss an eine Reaktion wiederum eine aktuelle Druckerfassung, um insbesondere die Auswirkung der Reaktion auf das Erreichen des stabilen Zustands beurteilen zu können. Ergibt sich hier gegenüber dem stabilen Zustand eine Druckänderung von Null, so ist dieser erfolgreich durch die Reaktion wieder herbeigeführt worden. Besteht hingegen immer noch eine Druckänderung, so wird erneut eine Reaktion eingeleitet.

In vorteilhafter Ausgestaltung der Erfindung ist mindestens eine Reaktion in Abhängigkeit der Druckänderung parametrisierbar, sodass geringe Druckänderungen insbesondere andere oder schwächere Reaktionen hervorrufen als hohe Druckänderungen.

In vorteilhafter Ausgestaltung der Erfindung erfolgen auch während einer laufenden Reaktion aktuelle Druckerfassungen, um die Reaktion in Abhängigkeit der Druckänderung parametrisieren oder beenden zu können.

In vorteilhafter Ausgestaltung der Erfindung wird das Entscheidungskriterium zur Einleitung einer Reaktion mit einem Hysterese-Verhalten implementiert, um Oszillationen um den stabilen Zustand herum vorzubeugen.

In vorteilhafter Ausgestaltung der Erfindung erfolgt nach jedem Dosierungsschritt eine aktuelle Druckerfassung, um nach der Dosierung das Erreichen des stabilen Zustands sicherzustellen.

In vorteilhafter Ausgestaltung der Erfindung erfolgen auch während einer laufenden Dosierung aktuelle Druckerfassungen, um eine möglicherweise notwendige, der Dosierung nachfolgende Reaktion vorherzusehen oder die Präzision der Dosierung zu verbessern.

In einigen Ausführungsformen der Erfindung kann der stabile Zustand nach der aktuellen Druckerfassung neu definiert werden, insbesondere aber nicht ausschließlich, um verschiedenen Drift-Effekten von Sensorik und Umgebungsparametern sowie um den Folgen der Volumenänderungen der Flüssigkeit im Behälter infolge von Dosierungen Rechnung zu tragen.

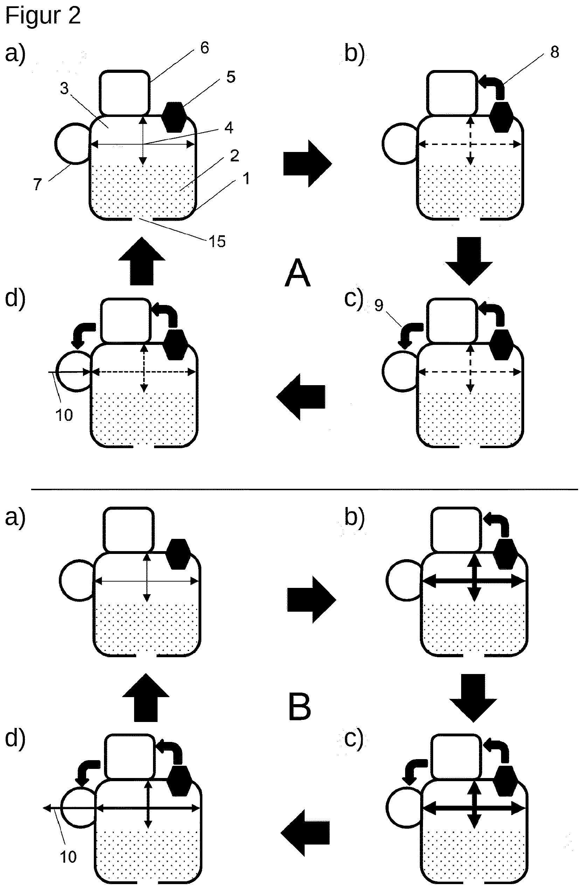

Figur 2 zeigt eine schematische Blockdarstellung des erfindungsgemäßen Verfahrens unter Verwendung einer erfindungsgemäßen Vorrichtung. Figur 2 ist unterteilt in die zwei Bereiche A (Figur 2-A) und B (Figur 2-B, wobei in Figur 2-A eine negative Druckänderung (z.B. bedingt durch Abkühlung der Flüssigkeit **2)** und in Figur 2-B eine positive Druckänderung (z.B. bedingt durch Erwärmung der Flüssigkeit **2)** erfasst und durch eine Reaktion **9** korrigiert wird.

Erfindungsgemäß umfasst die Vorrichtung einen Behälter **1,** welcher mit mindestens einer Flüssigkeit **2** zumindest teilweise gefüllt ist und der über eine Öffnung **15** verfügt, welche erfindungsgemäß permanent geöffnet ist. In einigen Ausgestaltungsformen der Erfindung befindet sich zur Zeit des Ablaufs des erfindungsgemäßen Verfahrens permanent Flüssigkeit **2** über der Öffnung **15.**

In vorteilhafter Ausgestaltung der Erfindung umfasst der Behälter **1** zudem mindestens eine Gasphase **3.** Durch mindestens einen Drucksensor **5** wird mindestens ein Druck **4** innerhalb des Behälters **1** erfasst. In vorteilhafter Ausgestaltung der Erfindung wird dabei zumindest ein Druck **4** der Gasphase **3** durch mindestens einen Drucksensor **5** erfasst.

Die erfindungsgemäße Vorrichtung umfasst ferner mindestens eine Regelungseinheit **6** und mindestens eine Fluidtransportvorrichtung **7,** wobei diese sowohl aktiv als auch passiv ausgeführt sein kann, insbesondere als Pumpe oder Ventil.

Figur 2 verdeutlicht die Anwendung des erfindungsgemäßen Verfahrens durch eine erfindungsgemäße Vorrichtung anhand vier exemplarischer Teilschritte a, b, c und d, dargestellt in jedem der Bereiche A und B. Die Initialisierung des Verfahrens ist dabei nicht dargestellt, sondern Teilschritt 2-A-a) bzw. 2-B-a) zeigt jeweils den stabilen Zustand des gesamten Systems nach Abschluss der initialen Druckerfassung. Auch auf die Darstellung des Dosierungsschrittes wurde verzichtet, da mögliche Dosierungsschritte aus dem stabilen Zustand heraus (z.B. durch Ventilöffnung oder durch Fluidförderung oder -bewegung mittels Pumpvorrichtungen) dem Fachmann geläufig sind.

Durch das Wirken mindestens eines Einflussfaktors (z.B. Temperatur) kommt es in Figur 2-A-b zu einem Druckabfall (gestrichelte Pfeile) und in Figur 2-B-b zu einem Druckanstieg (fette Pfeile). Die jeweilige Änderung des Drucks **4** im Vergleich zum Druck **4** des stabilen Zustands in Figur 2-A-a beziehungsweise Figur 2-B-a wird durch den Drucksensor **5** erfasst, und in Form von Druckwerten **8** an die Regelungseinheit **6** übertragen. Diese Regelungseinheit löst daraufhin, wie in Figur 2-A-c und Figur 2-B-c gezeigt, mindestens eine Reaktion **9** aus, welche insbesondere der Druckveränderung entgegenwirkt. Derartige Reaktionen **9** umfassen in vorteilhafter Ausgestaltung der Erfindung gesteuerte Aktionen der Fluidtransportvorrichtung **7,** insbesondere, aber nicht ausschließlich das Öffnen oder Schließen mindestens eines Ventils, das Hineinfördern mindestens eines Fluides in den Behälter **1** oder das Hinausfördern mindestens eines Fluides aus dem Behälter **1** durch mindestens eine Pumpe oder Kombinationen aus Pumpe und Ventil. Infolge der jeweiligen Reaktion **9** erfolgt in vorteilhafter Ausgestaltung der Erfindung ein Druckausgleich **10** und der Druck **4** im Behälter **1** normalisiert sich wieder hin zum stabilen Zustand, welcher nach Beendigung mindestens einer Reaktion **9** erreicht ist. Andernfalls setzt nach der nächsten Erfassung des Drucks **4** durch Drucksensor **5,** wie im Verfahrensflussdiagramm von Figur 1 erläutert, mindestens eine weitere Reaktion **9** ein.

Erfindungsgemäß verhindert die Reaktion **9,** insbesondere aber nicht ausschließlich durch den Druckausgleich **10** im Behälter **1** und die damit einhergehende Wiederherstellung des stabilen Zustands eine Umverteilung der Flüssigkeit **2** innerhalb oder außerhalb des Behälters **1.** So wird in Figur 2-A ein Einsaugen der Flüssigkeit **2** in den Behälter **1** verhindert, wodurch einer, durch das entstehende Totvolumen begründeten, nachteiligen Herabsetzung der Dosierpräzision entgegengewirkt wird. Im Gegensatz dazu wird in Figur 2-B ein Herausdrücken von Teilen der Flüssigkeit **2** aus dem Behälter **1** durch die Öffnung **15** verhindert, wodurch einem ungewollten, nachteiligen Dosiervorgang entgegengewirkt wird.

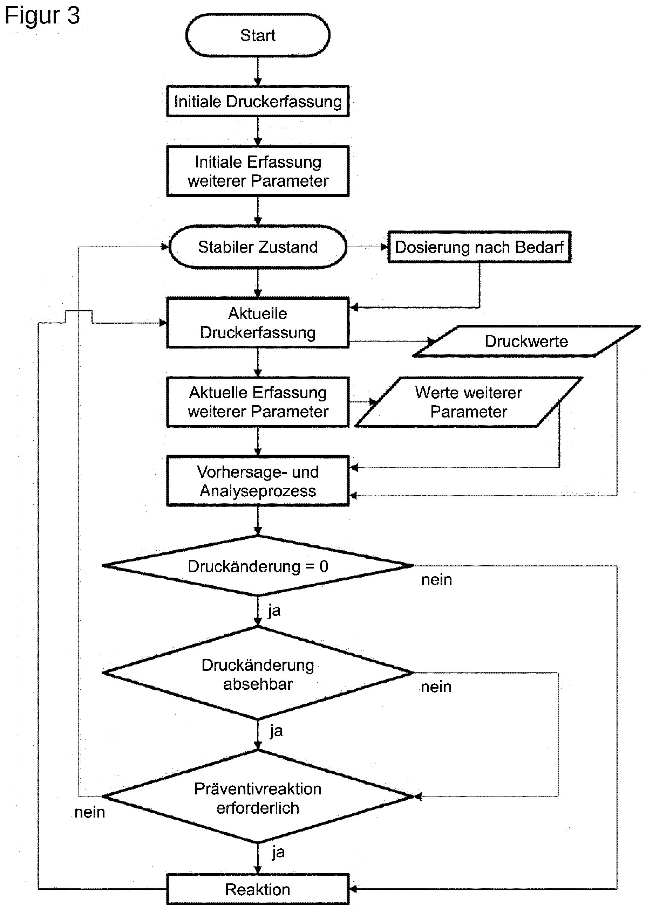

Figur 3 zeigt ein schematisches Flussdiagramm einer Ausführungsform des erfindungsgemäßen Verfahrens mit zusätzlicher Erfassung weiterer Parameter sowie mit Vorhersage- und Analyseverfahren und präventiver Reaktion. Zu Beginn des erfindungsgemäßen Verfahrens hat die Vorrichtung einen Start-Zustand. Aus dem Startzustand erfolgt mindestens eine initiale Druckerfassung. Weiterhin erfolgt die initiale Erfassung weiterer Parameter durch geeignete Sensoren. Sämtliche initialen Druck- und Parametererfassungen können erfindungsgemäß vollständig oder teilweise parallel oder sequentiell erfolgen.

Nach Abschluss der Initialisierungsphase befindet sich das System aus erfindungsgemäßer Vorrichtung und dem Behälterinhalt in einem stabilen Zustand. In vorteilhafter Ausgestaltung der Erfindung erfolgen alle weiteren Verfahrensschritte entweder aus dem stabilen Zustand heraus, oder haben das Ziel, den stabilen Zustand zu erhalten oder wiederherzustellen.

Erfindungsgemäß erfolgt je nach Bedarf die Dosierung von Flüssigkeit aus dem Behälter aus dem stabilen Zustand heraus. Der Bedarf ergibt sich dabei insbesondere, aber nicht ausschließlich aus zuvor festgelegten oder programmierten Dosierungsvorschriften, Gradienten, aus Interaktionen des Nutzers mit der erfindungsgemäßen Vorrichtung oder mit Vorrichtungen, die die erfindungsgemäße Vorrichtung steuern sowie aus Steuerbefehlen anderer Vorrichtungen, welche den Bedarf zur Dosierung anhand messbarer Parameter bestimmen.

Sowohl nach jedem Dosiervorgang, als auch direkt aus dem stabilen Zustand heraus erfolgt in regelmäßigen oder unregelmäßigen Abständen eine Überprüfung des aktuellen Zustands, um den stabilen Zustand des Systems aufrechtzuerhalten. Erfindungsgemäß wird dazu mindestens eine aktuelle Druckerfassung durchgeführt. Weiterhin erfolgt in vorteilhafter Ausgestaltung der Erfindung mindestens eine aktuelle Erfassung weiterer Parameter, wobei sämtliche aktuellen Druck- und Parametererfassungen erfindungsgemäß vollständig oder teilweise parallel oder sequentiell erfolgen können. Die resultierenden aktuellen Werte des Drucks und weiterer Parameter werden parallel zur oder im Anschluss an ihre Erfassung von einem Vorhersage- und Analyseprozess verarbeitet. In vorteilhafter Ausgestaltung der Erfindung erfolgt dies durch mindestens eine Regelungseinheit, welche dann insbesondere, aber nicht ausschließlich durch einen Rechner gebildet wird.

Erfindungsgemäße Vorhersage- und Analyseprozesse implementieren vorteilhaft insbesondere aber nicht ausschließlich Vorhersagemethoden wie Kalman-Filter oder anderweitige bayessche Schätzverfahren, Regressions- und Extrapolationsmethoden sowie Optimierungsmethoden zum Kurvenfitting und zur Modellverbesserung.

In vorteilhafter Ausgestaltung der Erfindung umfasst der Vorhersage- und Analyseprozess den Aufbau, die Parametrisierung und die Optimierung mindestens eines Modells für den aktuellen Zustand des Systems sowie zur Vorhersage zukünftiger Zustände des Systems, wobei neben dem Druck auch weitere Parameter vorteilhaft in die Modellausgestaltung und Optimierung einbezogen werden. In einigen Ausführungsformen der Erfindung werden dabei nicht nur aktuelle, sondern auch zeitlich zurückliegende Werte in die Berechnungen einbezogen.

Erfindungsgemäß ergibt sich aus dem Vorhersage- und Analyseprozess ein umfassendes Bild des aktuellen Systemzustands, welches Grundlage zur Entscheidung über die Einleitung von Reaktionen ist. Dazu werden, je nach verwendeten Parametern und Modellen, verschiedene Kriterien überprüft, die sich sowohl auf einzelne aktuelle, zurückliegende oder zukünftige Werte und Parameter beziehen können, oder die Informationen aus aktuellen, zurückliegenden oder zukünftigen Zuständen mindestens eines Modells ziehen und mittels kombinierter oder stärker abstrahierter oder komplexer Kriterien die Notwendigkeit der Einleitung einer Reaktion evaluieren. Erfindungsgemäß erfolgt die Evaluation von Kriterien zur Einleitung einer Reaktion vollständig oder teilweise parallel oder sequentiell.

In einigen Ausführungsformen der Erfindung kann im Falle einer erfassten Änderung mindestens eines kritischen Parameters oder Drucks auch der Vorhersage- und Analyseprozess übersprungen werden, um schneller eine Reaktion einzuleiten. Ebenso kann in einem solchen Fall in einigen Ausführungsformen der Erfindung die laufende Erfassung von Parametern abgebrochen werden, um schneller eine Reaktion einzuleiten. Ein Beispiel dafür ist die Erfassung einer starken Beschleunigung des Systems nach oben (z.B. beim Gehen oder beim Anheben des Systems), welche infolge der Trägheit der Flüssigkeit im Behälter zu einem Entweichen von Teilen der Flüssigkeit aus dem Behälter durch die Öffnung führen könnte. Durch direktes einleiten einer Reaktion (beispielsweise teilweises oder vollständiges Absaugen der Gasphase über der Flüssigkeit durch eine Pumpe als Fluidtransportvorrichtung) kann dieser nachteiligen Auswirkung der Beschleunigung des Systems als Einflussfaktor entgegengewirkt werden.

In vorteilhafter Ausgestaltung der Erfindung erfolgen auch während einer laufenden Dosierung aktuelle Druckerfassungen und aktuelle Erfassungen weiterer Parameter, deren Werte ebenfalls durch mindestens einen Analyse- und Vorhersageprozess verarbeitet werden können, um die möglicherweise notwendige, der Dosierung nachfolgende Reaktionen vorherzusehen oder die Präzision der Dosierung zu verbessern.

Figur 3 zeigt exemplarisch die sequentielle Evaluation dreier Kriterien unterschiedlicher Komplexität. In Analogie zu Figur 1 wird zunächst überprüft, ob eine Druckänderung erfasst wurde. Ist dies der Fall, so wird eine Reaktion eingeleitet. Andernfalls wird in vorteilhafter Ausgestaltung der Erfindung evaluiert, ob in naher oder ferner Zukunft eine Druckänderung absehbar ist. Je nach Ergebnis dieser Evaluation kann dann entschieden werden, ob eine Reaktion als Präventivreaktion erforderlich ist, oder ob der stabile Zustand erreicht oder erhalten wurde. Typische Beispiele für eine Präventivreaktion sind insbesondere alle Reaktionen, die sich auf Nutzereingaben oder andere sich planmäßig oder modellierbar verhaltende Daten beziehen. So kann erfindungsgemäß auf die sinngemäße Nutzereingabe "System wird gleich getragen" durch definiertes Absaugen der Gasphase über der Flüssigkeit im Behälter die Flüssigkeit präventiv tiefer in den Behälter gezogen werden, um so Fehldosierungen oder sonstigem Verlust von Flüssigkeit durch die Öffnung infolge der Erschütterungen beim Gehen vorzubeugen.

In vorteilhafter Ausgestaltung der Erfindung erfolgen auch während einer laufenden Reaktion aktuelle Druckerfassungen und Erfassungen weiterer Parameter, um die Reaktion in Abhängigkeit der Druckänderung parametrisieren oder beenden zu können.

Erfindungsgemäß werden nach jeder Reaktion erneut der aktuelle Druck sowie weitere aktuelle Parameter erfasst, um das Erreichen oder die Aufrechterhaltung des stabilen Zustandes zu überprüfen.

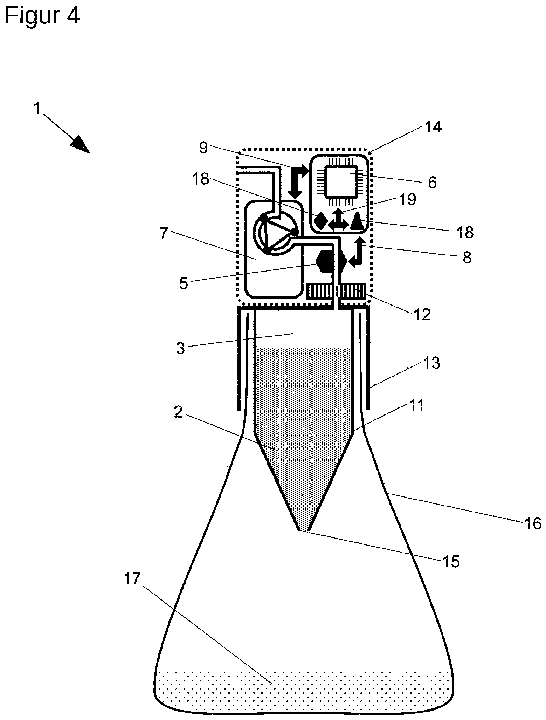

Figur 4 zeigt eine schematische Darstellung einer erfindungsgemäßen Vorrichtung zur Durchführung des erfindungsgemäßen Verfahrens zur Kultivierung von Zellen. Vorteilhaft ist die Erfindung für die Kultivierung von Zellen, da sie die gesteuerte Zugabe von Substanzen zur Kulturbrühe erlaubt, insbesondere aber nicht ausschließlich die Zugabe von Nährstoffen, pH-Stellmitteln, Antischaum und Induktoren. Dabei ist die erfindungsgemäße Vorrichtung unter Anwendung des erfindungsgemäßen Verfahrens vorteilhaft geeignet, die Vorteile und die Einfachheit eines permanent nach unten hin geöffneten Dosiersystems auch unter solchen Bedingungen zu gewährleisten, unter denen es nach dem Stand der Technik zu Fehldosierungen, Flüssigkeitsverlust oder einem Verlust der Dosierungspräzision kommen würde.

In der dargestellten Ausführungsform der Erfindung erfolgt die Dosierung hinein in einen Schüttelkolben **16** als Gefäß, welcher z.B. mit Kulturbrühe **17** befüllt ist. Die erfindungsgemäße Vorrichtung wird nach dem Befüllen auf das Gefäß **16** aufgesetzt und mit dieser fest verbunden. Die Vorrichtung dient dort der Dosierung und Aufbewahrung der zu dosierenden Flüssigkeit.

In vorteilhafter Ausgestaltung der Erfindung umfasst der Behälter **1** mehrere Teilen, nämlich ein Flüssigkeitsreservoir **11** und eine Dosiervorrichtung **14.** Das Flüssigkeitsreservoir **11** als Bestandteil des Behälters **1** besitzt eine permanent offene Öffnung **15** und ist zumindest teilweise gefüllt mit Flüssigkeit **2** und einer Gasphase **3,** insbesondere Luft.

Die Befüllung des Flüssigkeitsreservoirs **11** mit Flüssigkeit **2** erfolgt in einigen Ausführungsformen der Erfindung über die Öffnung **15,** entweder unter Verwendung der Dosiervorrichtung **14,** oder unter Zuhilfenahme anderer geeigneter Vorrichtungen zur Förderung von Fluiden.

Das Flüssigkeitsreservoir 11 ist über einen Sterilfilter **12** als Sterilitätsbarriere mit der Dosiervorrichtung **14** gasdicht verbunden. In vorteilhafter Ausgestaltung der Erfindung ist der Raum unterhalb des Sterilfilters **12** steril, wohingegen die zur erfindungsgemäßen Bewegung oder Verdrängung der Flüssigkeit **2** eingesetzte Luft oberhalb des Sterilfilters **12** unsteril sein darf, insbesondere da die Luft nach dem Durchgang durch den Sterilfilter **12** selbst steril ist. Eine solche Ausführung ist vorteilhaft, um den Sterilitätsansprüchen im Anwendungsbereich der Kultivierung von Zellen Rechnung zu tragen, da somit das Flüssigkeitsreservoir **11** als austauschbares, vorsterilisiertes Einwegteil verwendet werden kann, während die Dosiervorrichtung **14** immer unsteril sein darf, was insbesondere hinsichtlich Konstruktion, Materialzusammensetzung und Handhabbarkeit vorteilhaft ist.

In vorteilhafter Ausgestaltung der Erfindung umfasst das Flüssigkeitsreservoir **11** auch den Deckel **13** des Gefäßes **16,** sodass Handhabung und Sterilitätserhaltung durch die Fertigung aus einem Stück (z.B. durch Spritzguss) vereinfacht werden.

In vorteilhafter Ausgestaltung der Erfindung befindet sich im unsterilen Teil der Vorrichtung mindestens ein Drucksensor **5** als Bestandteil der Dosiervorrichtung **14.** Ebenso umfasst die Dosiervorrichtung **14** in vorteilhafter Ausgestaltung der Erfindung weitere Sensoren **18** zur Erfassung weiterer Parameter, insbesondere aber nicht ausschließlich Nutzereingaben, Temperatur, Beschleunigung, Flüssigkeitsstand, Position und Lage. Die Druckwerte **8** und die Werte weiterer Parameter **19** werden erfindungsgemäß an mindestens eine Regelungseinheit **6** übermittelt, welche in vorteilhafter Ausgestaltung der Erfindung insbesondere als Rechner ausgeführt ist und welche die Einleitung, Überwachung, Parametrisierung und Beendigung von Reaktionen **9** durchführt.

Dosierungsprozesse und Reaktionen erfolgen in vorteilhafter Ausgestaltung der Erfindung durch dieselbe Fluidtransportvorrichtung **7.** Ebenso erfolgt auch die Regelung dieser Dosierungsprozesse und Reaktionen in vorteilhafter Ausgestaltung der Erfindung durch dieselbe Regelungseinheit **6.**

In Figur 4 ist die Fluidtransportvorrichtung **7** als Pumpe, insbesondere als Peristaltikpumpe ausgeführt, welche Luft als Gasphase **3** in den Behälter **1** hinein oder aus dem Behälter 1 heraus befördert. Insofern kann die Fluidtransportvorrichtung **7** noch Bestandteil des Behälters **1 sein,** welcher in der dargestellten Ausführungsform das Flüssigkeitsreservoir **11,** den Sterilfilter **12,** den Drucksensor **5,** Teile der Fluidtransportvorrichtung **7** sowie alle nicht weiter genannten Verbindungsstücke zwischen diesen Einheiten umfasst.

Das erfindungsgemäße Verfahren wird durch die in Figur 4 dargestellte erfindungsgemäße Vorrichtung folgendermaßen umgesetzt. Zum Zweck der Dosierung einer Flüssigkeit **2** wird das Flüssigkeitsreservoir **11** mit Flüssigkeit **2** befüllt, mit der Dosiervorrichtung **14** verbunden und dann mittels des in das Flüssigkeitsreservoir **11** integrierten Deckels **13** auf den mit Kulturbrühe **17** befüllten Gefäß **16** aufgesteckt.

Die initiale Erfassung des Drucks und weiterer Parameter wird dabei entweder durch eine Nutzereingabe nach Abschluss der Installation der Vorrichtung auf dem Gefäß **16** ausgelöst, oder aber sie läuft bereits von Beginn an und wird erst dann einen stabilen Zustand erreichen, wenn die Installation abgeschlossen ist und der Aufbau aus Gefäß **16** und erfindungsgemäßer Vorrichtung unbewegt steht.

Ab dem Erreichen des stabilen Zustands werden aus diesem Zustand gemäß zuvor getätigter oder nachträglicher Nutzereingaben Dosierungen durch die Regelungseinheit **6** eingeleitet und von der Peristaltikpumpe als Fluidtransportvorrichtung **7** durchgeführt. Dazu wird Luft als Gasphase **3** durch den Sterilfilter **12** in das Flüssigkeitsreservoir **11** gedrückt und verdrängt dadurch Teile der Flüssigkeit **2,** welche infolgedessen durch die Öffnung **15** in die Kulturbrühe **17** abgegeben werden. Nach Abschluss oder parallel zu einer jeden Dosierung und auch sonst in regelmäßigen oder unregelmäßigen Abständen erfasst der Drucksensor **5** den Druck der Gasphase **3** und übermittelt die resultierenden Druckwerte **8** an die Regelungseinheit **6.** Ebenso werden durch weitere Sensoren **18** weitere Parameter erfasst und die resultierenden Werte **19** zur Regelungseinheit **6** übermittelt.

Erfindungsgemäß analysiert die Regelungseinheit **6** sämtliche eingegangenen Werte **8/19** und berechnet aus ihnen mindestens ein Modell des aktuellen Zustands sowie gegebenenfalls zukünftiger Zustände. Mindestens ein Modell wird dann zur Bewertung des aktuellen Zustands anhand geeigneter Kriterien evaluiert und Reaktionen **9** zur Aufrechterhaltung oder Wiederherstellung des stabilen Zustands werden je nach Evaluationsresultat eingeleitet. Im in Figur 4 dargestellten Ausführungsbeispiel erfolgen die Reaktionen über die Peristaltikpumpe als Fluidtransportvorrichtung **7** durch die Förderung von Luft als Gasphase **3.** Nachfolgend sind einige typische Zustände oder Werte gemeinsam mit einer beispielhaften geeigneten Reaktion aufgeführt.

Wurde ein zu hoher Druck gemessen, so befördert die Fluidtransportvorrichtung **7** je nach Druckdifferenz eine bestimmte Menge der Gasphase **3** aus dem Behälter **1** heraus, um den Druck des stabilen Zustands wiederherzustellen. Wurde ein zu niedriger Druck gemessen, so befördert die Fluidtransportvorrichtung **7** je nach Druckdifferenz eine bestimmte Menge der Gasphase **3** in den Behälter **1** hinein, um den Druck des stabilen Zustands wiederherzustellen.

Wurde eine vorhersehbare Bewegung des Behälters **1** durch eine Nutzereingabe erfasst, so befördert die Fluidtransportvorrichtung **7** eine bestimmte Menge der Gasphase **3** aus dem Behälter **1** heraus, um die Flüssigkeit **2** nach oben in den Behälter **1** zu ziehen und somit einem beschleunigungsbedingten, nachteiligen Flüssigkeitsverlust vorzubeugen. Ebenso befördert die Fluidtransportvorrichtung **7** eine bestimmte Menge der Gasphase **3** in den Behälter **1** zurück, sobald durch Nutzereingabe das Ende der vorhersehbaren Bewegung des Behälter **1** erreicht ist.

Wird durch mindestens einen Temperatursensor eine Änderung der Umgebungstemperatur erfasst, so ist auch eine entsprechende Änderung der Temperatur der Flüssigkeit **2** und des Behälters **1** vorhersehbar. Der thermodynamisch daraus resultierenden vorhersehbaren Druckänderung im Behälter kann durch eine Reaktion **9** mit präventivem Druckausgleich (Hinein- oder Hinausbeförderung von Gasphase **3** in den oder aus dem Behälter **1)** durch die Fluidtransportvorrichtung **7** entgegengewirkt werden.

### Bezugszeichenliste

Zur jeweiligen Interpretation der Bezugszeichen sind Beschreibung und Ansprüche zu beachten.

| | |
|---|---|
| **1** | Behälter |
| **2** | Flüssigkeit |
| **3** | Gasphase, z.B. Luft |
| **4** | Druck |
| **5** | Drucksensor |
| **6** | Regelungseinheit |
| **7** | Fluidtransportvorrichtung, passiv oder aktiv |
| **8** | Druckwerte |
| **9** | Reaktion |
| **10** | Druckausgleich |
| **11** | Flüssigkeitsreservoir |
| **12** | Sterilfilter |
| **13** | Deckel |
| **14** | Dosiervorrichtung |
| **15** | Permanent offene Öffnung |
| **16** | Gefäß |
| **17** | Kulturbrühe |
| **18** | Weitere Sensoren zur Erfassung weiterer Parameter |
| **19** | Werte weiterer Parameter |

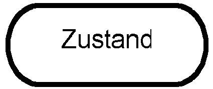

Zustand einer Vorrichtung, welche das erfindungsgemäße Verfahren durchführt 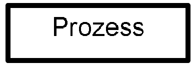
Prozess als durchzuführender Schritt des erfindungsgemäßen Verfahrens 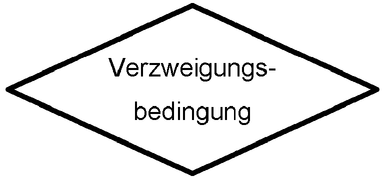
Verzweigungsbedingung im Verlauf des erfindungsgemäßen Verfahrens. Angegeben ist das jeweilige Verzweigungskriterium. Die abgehenden 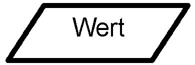
Wert als Datenpunkt, Datensammlung oder sonstige manifeste Information im Verlauf des erfindungsgemäßen Verfahrens 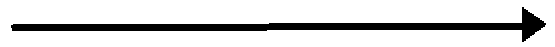
Ablaufrichtung des erfindungsgemäßen Verfahrens von mindestens einem Zustand, Prozess, Wert oder einer Verzweigungsbedingung zu mindestens einem Zustand, Prozess, Wert oder einer Verzweigungsbedingung

## Patentansprüche

1. Verfahren zur Dosierung und Aufbewahrung von Flüssigkeiten mittels eines permanent offenen Behälters (1),
wobei der Behälter (1) ein Flüssigkeitsreservoir (11) umfasst, in welchem mindestens eine Flüssigkeit (2) zur Aufbewahrung enthalten ist,
wobei der Behälter (1) mindestens eine Öffnung (15) zur dosierten Abgabe der Flüssigkeit (2) aus dem Flüssigkeitsreservoir (11) aufweist,
wobei die Öffnung (15) permanent unverschlossen ist,
wobei Einflussfaktoren mindestens eine Änderung des Drucks (4) im Behälter (1) hervorrufen,
wobei Einflussfaktoren das aus Behälter (1) und Flüssigkeit (2) gebildete System in einen unstabilen Zustand versetzen,
wobei an mindestens einem Ort im Behälter (1) der Druck (4) erfasst wird, und
wobei in Abhängigkeit mindestens einer erfassten Änderung des Drucks (4) mindestens eine Reaktion (9) eingeleitet wird, welche geeignet ist, das System aus Behälter (1) und Flüssigkeit (2) in einem stabilen Zustand zu halten oder diesen stabilen Zustand wiederherzustellen,
**dadurch gekennzeichnet,**
**dass** der Behälter (1) neben dem Flüssigkeitsreservoir (11) eine Dosiervorrichtung (14) aufweist, wobei zwischen dem Flüssigkeitsreservoir (11) und der Dosiervorrichtung (14) eine Sterilitätsbarriere eingebracht ist.

2. Verfahren nach dem vorherigen Anspruch,
**dadurch gekennzeichnet,**
**dass** durch die mindestens eine Reaktion mindestens einer Änderung der Verteilung der Flüssigkeit entgegengewirkt oder vorgebeugt wird.

3. Verfahren nach einem der vorherigen Ansprüche,
**dadurch gekennzeichnet,**
**dass** innerhalb des Flüssigkeitsreservoirs (11) eine Gasphase (3) ausgebildet ist und dass mindestens ein Drucksensor (5) mindestens einen Druck (4) der Gasphase (3) erfasst.

4. Verfahren nach einem der vorherigen Ansprüche,
**dadurch gekennzeichnet,**
**dass** mindestens ein weiterer Parameter (18) erfasst und in den Entscheidungsprozess über das Einleiten mindestens einer Reaktion (9) einbezogen wird.

5. Verfahren nach einem der vorherigen Ansprüche,
**dadurch gekennzeichnet,**
**dass** mögliche zukünftige Zustände auf Basis erfasster Werte (8) des Druckes (4) oder erfasster Werte weiterer Parameter (19) vorhergesagt und zur Evaluierung der Notwendigkeit einer Einleitung mindestens einer Präventivreaktion herangezogen werden.

6. Verfahren nach einem der vorherigen Ansprüche,
**dadurch gekennzeichnet,**
**dass** die gezielte Bewegung der Flüssigkeit (2) im Behälter (1) zum Zweck der Dosierung oder der Durchführung von Reaktionen (9) über die Bewegung einer Gasphase (3) erfolgt.

7. Verfahren nach einem der vorherigen Ansprüche,
**dadurch gekennzeichnet,**
**dass** Dosierungen und Reaktionen eine gemeinsame Regelungseinheit (6) gesteuert werden.

8. Verfahren nach einem der vorherigen Ansprüche,
**dadurch gekennzeichnet,**
**dass** Dosierungen und Reaktionen durch eine gemeinsame Fluidtransportvorrichtung (7) durchgeführt werden.

9. Verfahren nach einem der vorherigen Ansprüche,
**dadurch gekennzeichnet,**
**dass** der Behälter (1) derart zu einem Gefäß (16), insbesondere einem Schüttelkolben (16), angeordnet ist, dass die abgegebene Flüssigkeit (2) durch die Öffnung (15) in das Gefäß (16) gelangt.

10. Verfahren nach dem vorherigen Anspruch,
**dadurch gekennzeichnet,**
**dass**
- das Erfassen des Drucks (4) und/oder
- das Einleiten der Reaktion und/oder
- das Abgeben der Flüssigkeit
erfolgt, während das Gefäß (16) geschüttelt wird.

11. Verfahren nach dem vorherigen Anspruch,
**dadurch gekennzeichnet,**
**dass** das Behälter (1) fest mit dem Gefäß (16) verbunden ist, insbesondere dass der Behälter (1), insbesondere dauerhaft und/oder während eines Schüttelvorgangs, das Gefäß (16) verschließt.

12. Vorrichtung, die zur Durchführung des Verfahrens nach einem der vorherigen Ansprüche eingerichtet ist,
wobei die Vorrichtung mindestens einen Behälter (1), insbesondere mit einem Flüssigkeitsreservoir (11), umfasst, welcher mindestens teilweise mit Flüssigkeit (2) gefüllt ist und über mindestens eine permanente Öffnung (15) verfügt, und wobei die Vorrichtung mindestens einen Drucksensor (5), mindestens eine Fluidtransportvorrichtung (7) und eine Regelungseinheit (6) umfasst, welche die mindestens eine Fluidtransportvorrichtung (7) zum Zweck der Durchführung von Reaktionen (9) oder Dosierungen ansteuert,
**dadurch gekennzeichnet,**
**dass** in der Fluidverbindung zwischen dem Drucksensor (5) und dem Flüssigkeitsreservoir (11) und/oder in der Fluidverbindung zwischen der Fluidtransportvorrichtung (7) und dem Flüssigkeitsreservoir (11) eine Sterilitätsbarriere (12) angeordnet ist.

13. Vorrichtung nach dem vorherigen Anspruch,
**dadurch gekennzeichnet,**
**dass** die Regelungseinheit (6) eingerichtet ist, ein Verfahren nach einem der vorherigen Ansprüche 1 bis 11 zu steuern.

14. Verwendung einer Vorrichtung nach einem der Ansprüche 12 bis 13 zur Durchführung eines Verfahrens nach einem der Ansprüche 1 bis 11.

15. Verwendung nach dem vorherigen Anspruch,
wobei das Flüssigkeitsreservoir (11) von der Vorrichtung, insbesondere von dem Drucksensor (5) und/oder von der Fluidtransportvorrichtung (7), abgetrennt wird und vom Gefäß (16) entfernt wird,
anschließend ein neues Flüssigkeitsreservoir (11) an die Vorrichtung angeschlossen wird und mit dem Gefäß (16) oder einem anderen Gefäß (16) derart angeordnet wird, dass die abzugebende Flüssigkeit (2) aus dem neuen Flüssigkeitsreservoir (11) durch die Öffnung (15) in das Gefäß (16) gelangen kann.

## Claims

1. Method for dosing and storage of liquids by means of a permanently open container (1), wherein the container (1) comprises a fluid reservoir (11) in which at least one fluid (2) is held for storage,
wherein the container (1) has at least one opening (15) for the dosed administration of the fluid (2) out of the fluid reservoir (11),
wherein the opening (15) is permanently unsealed,
wherein influencing factors cause at least one change in the pressure (4) in the container (1), wherein influencing factors transfer the system formed by container (1) and fluid (2) into an unstable state,
wherein the pressure (4) is recorded in at least one place in the container (1), and
wherein at least one reaction (9) is initiated depending on at least one recorded change of pressure (4), which reaction is suitable for retaining the system of container (1) and fluid (2) in a stable state or to restore this stable state,
**characterised in that**
besides the fluid reservoir (11), the container (1) has a dosing device (14) wherein a sterility barrier is interposed between the fluid reservoir (11) and the dosing device (14).

2. Method according to the previous claim,
**characterised in that**
at least one change in the distribution of the fluid is counteracted or prevented by at least one reaction.

3. Method according to any one of the preceding claims,
**characterised in that**
a gas phase (3) is formed inside the fluid reservoir (11) and that at least one pressure sensor (5) records at least one pressure (4) of the gas phase (3).

4. Method according to any one of the preceding claims,
**characterised in that**
at least one further parameter (18) is recorded and is involved in the decision process about the initiation of at least one reaction (9).

5. Method according to any one of the preceding claims,
**characterised in that**
possible future states based on recorded values (8) of the pressure (4) or recorded values of other parameters (19) are predicted and are invoked to evaluate the necessity of initiating at least one preventive reaction.

6. Method according to any one of the preceding claims,
**characterised in that**
the aimed-for movement of the fluid (2) in the container (1) for the purpose of the dosing or the performance of reactions (9) takes place through the movement of a gas phase (3).

7. Method according to any one of the preceding claims,
**characterised in that**
dosing and reactions are controlled by a common control unit (6).

8. Method according to any one of the preceding claims,
**characterised in that**
dosing and reactions are performed through a common fluid transportation device (7).

9. Method according to any one of the preceding claims,
**characterised in that**
the container (1) is arranged on a vessel (16), in particular a shake flask (16), such that the delivered fluid (2) goes through the opening (15) into the vessel (16).

10. Method according to the preceding claim,
**characterised in that**
- the recording of the pressure (4) and/or
- the initiation of the reaction and/or
- the delivery of the fluid
is done while the vessel (16) is being shaken.

11. Method according to the preceding claim,
**characterised in that**
the container (1) is attached firmly to the vessel (16), in particular so that the container (1) seals the vessel (16) in particular permanently and/or during a shaking process.

12. Device which is set up to carry out the method according to any one of the preceding claims,
wherein the device comprises at least one container (1), in particular with a fluid reservoir (11), which is filled at least partly with fluid (2) and has at least one permanent opening (15) and wherein the device comprises at least one pressure sensor (5), at least one fluid transportation device (7) and one control unit (6) which controls the at least one fluid transportation device (7) with the aim of carrying out reactions (9) or dosing, **characterised in that**
a sterility barrier (12) is arranged in the fluid connection between the pressure sensor (5) and the fluid reservoir (11) and/or in the fluid connection between the fluid transportation device (7) and the fluid reservoir (11).

13. Device according to the preceding claim,
**characterised in that**
the control unit (6) is set up to control a method according to any one of the preceding claims 1 to 11.

14. Use of a device according to either of claims 12 to 13 to carry out a method according to any one of the preceding claims 1 to 11.

15. Use according to the preceding claim,
wherein the fluid reservoir (11) is separated from the device, in particular from the pressure sensor (5) and/or from the fluid transportation device (7) and
is at a distance from the vessel (16),
then a new fluid reservoir (11) is coupled to the device and is arranged with the vessel (16) or with another vessel (16) such that the fluid (2) to be dispensed can go out of the new fluid reservoir (11) through the opening (15) into the vessel (16).

## Revendications

1. Procédé de dosage et de conservation de liquides au moyen d'un récipient (1) ouvert en permanence,
le récipient (1) comprenant un réservoir de liquide (11), dans lequel au moins un liquide (2) est contenu pour être conservé,
le récipient (1) présentant au moins une ouverture (15) pour la distribution dosée du liquide (2) à partir du réservoir de liquide (11),
l'ouverture (15) étant ouverte en permanence,
les facteurs influents provoquant au moins un changement de la pression (4) dans le récipient (1),
les facteurs influents plaçant le système formé du récipient (1) et du liquide (2) dans un état instable,
la pression (4) étant saisie à au moins un endroit dans le récipient (1), et
en fonction d'au moins un changement détecté de la pression (4), au moins une réaction (9) étant lancée, qui est apte à maintenir le système formé du récipient (1) et du liquide (2) dans un état stable ou à rétablir cet état stable,
**caractérisé en ce que**
le récipient (1), outre le réservoir de liquide (11), présente un dispositif de dosage (14), une barrière stérile étant posée entre le réservoir de liquide (11) et le dispositif de dosage (14).

2. Procédé selon la revendication précédente,
**caractérisé en ce**
**qu'**au moyen de l'au moins une réaction au moins un changement de la distribution du liquide est contré ou évité.

3. Procédé selon l'une des revendications précédentes,
**caractérisé en ce**
**qu'**une phase gazeuse (3) se forme dans le réservoir de liquide (11) et **en ce qu'**au moins un capteur de pression (5) saisit au moins une pression (4) de la phase gazeuse (3).

4. Procédé selon l'une des revendications précédentes,
**caractérisé en ce**
**qu'**au moins un autre paramètre (18) est saisi et inclus dans le processus de décision concernant le lancement d'au moins une réaction (9).

5. Procédé selon l'une des revendications précédentes,
**caractérisé en ce**
**que** des états futurs possibles sont prévus sur la base de valeurs saisies (8) de la pression (4) ou de valeurs saisies d'autres paramètres (19) et utilisés pour l'évaluation de la nécessité d'un lancement d'au moins une réaction préventive.

6. Procédé selon l'une des revendications précédentes,
**caractérisé en ce**
**que** le mouvement ciblé du liquide (2) dans le récipient (1) a lieu à des fins de dosage ou de la réalisation de réactions (9) via le mouvement d'une phase gazeuse (3).

7. Procédé selon l'une des revendications précédentes,
**caractérisé en ce**
**que** les dosages et réactions sont commandés par une unité de régulation (6) commune.

8. Procédé selon l'une des revendications précédentes,
**caractérisé en ce**
**que** les dosages et réactions sont réalisés par un dispositif de transport de liquide (7) commun.

9. Procédé selon l'une des revendications précédentes,
**caractérisé en ce**
**que** le récipient (1) est disposé par rapport à un contenant (16), notamment un flacon agité (16), de telle sorte que le liquide (2) distribué parvient au contenant (16) par l'ouverture (15).

10. Procédé selon la revendication précédente,
**caractérisé en ce**
**que**
- la saisie de la pression (4) et/ou
- le lancement de la réaction et/ou
- la distribution du liquide
sont effectués alors que le contenant (16) est agité.

11. Procédé selon la revendication précédente,
**caractérisé en ce**
**que** le récipient (1) est relié fixement au contenant (16), notamment **en ce que** le récipient (1) ferme le contenant (16) notamment en permanence et/ou pendant qu'il est agité.

12. Dispositif conçu pour la réalisation du procédé selon l'une des revendications précédentes,
le dispositif comprenant au moins un récipient (1), notamment avec un réservoir de liquide (11), lequel est au moins partiellement rempli de liquide (2) et dispose d'au moins une ouverture (15) permanente, et le dispositif comprenant au moins un capteur de pression (5), au moins un dispositif de transport de liquide (7) et une unité de régulation (6), qui commande l'au moins un dispositif de transport de liquide (7) à des fins de réalisation de réactions (9) ou de dosages,
**caractérisé en ce**
**qu'**une barrière stérile (12) est disposée dans la communication fluidique entre le capteur de pression (5) et le réservoir de liquide (11) et/ou dans la communication fluidique entre le dispositif de transport de liquide (7) et le réservoir de liquide (11).

13. Dispositif selon la revendication précédente,
**caractérisé en ce**
**que** l'unité de régulation (6) est conçue pour commander un procédé selon l'une des revendications précédentes 1 à 11.

14. Utilisation d'un dispositif selon l'une des revendications 12 à 13 pour la réalisation d'un procédé selon l'une des revendication 1 à 11.

15. Utilisation selon la revendication précédente,
le réservoir de liquide (11) étant séparé du dispositif, notamment du capteur de pression (5) et/ou du dispositif de transport de liquide (7), et retiré du contenant (16),
puis un nouveau réservoir de liquide (11) étant raccordé au dispositif et disposé avec le contenant (16) ou un autre contenant (16) de telle sorte que le liquide (2) à distribuer à partir du nouveau réservoir de liquide (11) puisse parvenir au contenant (16) par l'ouverture (15).
